# EUROPEAN PATENT APPLICATION

(11) **EP 3 384 894 A1**
(43) Date of publication of application: **10.10.2018**
(21) Application number: 17179121.3
(22) Date of filing: 30.06.2017
(51) Int. Cl.: A61K 8/26, A61K 8/41, A61Q 19/00, A61K 8/02

(54) **LIQUID DROP RELEASING COSMETIC**

(30) Priority: 03.04.2017 KR 20170042863
(71) Applicant: Sunjin Beauty Science Co., Ltd., Ansan-si, Gyeonggi-do 15612 (KR)
(72) Inventor: LEE, Sung-Ho, 15612 Ansan-si, Gyeonggi-do (KR); PARK, Jang-Ho, 15612 Ansan-si, Gyeonggi-do (KR); YOON, Yang-Bae, 15612 Ansan-si, Gyeonggi-do (KR); SEONG, Deuk-Yong, 15612 Ansan-si, Gyeonggi-do (KR)
(74) Representative: Bockhorni & Brüntjen Partnerschaft Patentanwälte mbB

(57) **Abstract**

The present invention relates to a liquid drop releasing cosmetic material, and particularly to a cosmetic material of releasing liquid drop by rubbing, which comprises: (a) a card house structure having a space formed with a plate-shaped substance therein; and (b) liquid drop contained in the space of the card house structure.

A liquid drop releasing cosmetic material according to the present invention has an excellent visual effect and feeling of use by releasing liquid drop due to rubbing during use and fundamentally solves side effect and antipathy of users due to silicone polymer because it is free from a silicone polymer, and has excellent stability in a cosmetic material of a drop releasing structure.

## Description

### [Technical Field]

The present invention relates to a liquid drop releasing cosmetic material and more particularly, to a liquid drop releasing cosmetic material which has an excellent visual effect and feeling of use by releasing liquid drop due to rubbing during use and fundamentally solves side effect and antipathy of users due to silicone polymer because it is free from a silicone polymer, and has excellent stability in a cosmetic material of a drop releasing structure.

### [Background Art]

The contents described below merely provide background information related to the present invention and do not constitute the prior art.

Recently, a liquid drop releasing cosmetic material such as a water drop releasing cosmetic material has been preferred. The water releasing cosmetic material is a cosmetic material that bursts out water by rubbing during the use, and has a unique visual effect to the user and can have an excellent feeling for the user such as moistness and coolness, etc.

The water releasing structure component of the water releasing cosmetic material is highly stable in the cosmetic material, and since the water should be released well at rubbing, silicone polymers are mostly used as the structure of the water releasing component in the water releasing cosmetic material.

However, the silicone component used in the cosmetic material has been variously reported to be harmful to human body such as CMR (Carcinogenic, Mutagenic, Reprotoxic) and as a result, the use of cosmetics is limited starting in Europe.

Accordingly, it is urgently required to develop a cosmetic material having a water releasing effect without using a silicone component.

### [Disclosure]

### [Technical Problem]

The object of the present invention is to provide a liquid drop releasing cosmetic material and more particularly, to a liquid drop releasing cosmetic material which has an excellent visual effect and feeling of use by releasing liquid drop due to rubbing during use and fundamentally solves side effect and antipathy of users due to silicone polymer because it is free from a silicone polymer and has excellent stability in a cosmetic material of a drop releasing structure.

### [Technical Solution]

To achieve the object of the present invention, the present invention provides a cosmetic material of releasing liquid drop by rubbing, which comprises: (a) a card house structure having a space formed with a plate-shaped substance therein; and (b) liquid drop contained in the space of the card house structure.

The plate-shaped substance of the (a) may have an (+) electric charge on a surface thereof and an (-) electric charge in an edge thereof.

The plate-shaped substance of the (a) may be hectorite or bentonite modified with a cation.

The plate-shaped substance of the (a) may be distearmonium hectorite.

The plate-shaped substance of the (a) may further comprise: at least one oil selected from the group consisting of coco-caprylate/caprate, caprylic/capric triglyceride, dicaprylyl carbonate, myristyl myristate, dicaprylyl ether, cetyl palmitate, octyldodecanol, hexyldecanol, hexyldecyl stearate, cetyl palmitate, isostearyl isostearate, decyl oleate, hydrogenated vegetable oil, methylheptyl isostearate, nigella sativa seed oil, camellia oleifera seed oil and passiflora incarnata oil; and at least one emulsifier selected from the group consisting of polyglyceryl-4 isostearate (HLB 5), polyglyceryl-3 polyricinoleate (HLB 4), polyglyceryl-3 diisostearate (HLB 5.5), polyglyceryl-4 oleate (HLB 3), polyglyceryl-4 diisostearate/polyhydroxystearate/sebacate (HLB 5), diisostearoyl polyglyceryl-3 dimer dilinoleate (HLB 5), polyglyceryl-3 polyricinoleate (and) sorbitan isostearate (HLB 4.2), glyceryl laurate (HLB 5.2), sorbitan oleate (HLB 4.3) and sorbitan sesquioleate (HLB 3.7).

The card house structure of the (a) may comprise 5 to 20 parts by weight of a plate-shaped substance; 40 to 60 parts by weight of oil; and 30 to 50 parts by weight of an emulsifier.

The size of the card house structure having liquid drop in the space therein may be 10 to 60 µm in diameter.

The card house structure having a space formed with a plate-shaped substance therein of the (a) may be in an amount of 0.5 to 20% by weight of cosmetic composition.

Preferably, the liquid drop is water.

### [Advantageous Effects]

A liquid drop releasing cosmetic material according to the present invention has an excellent visual effect and feeling of use by releasing liquid drop due to rubbing during use and fundamentally solves side effect and antipathy of users due to silicone polymer because it is free from a silicone polymer and has excellent stability in a cosmetic material of a drop releasing structure.

### [Description of Drawings]

FIG. 1 is a schematic view showing that water is released from a water releasing cosmetic material.
FIG. 2 is a schematic view showing that a card house structure in which a liquid drop is contained according to an embodiment of the present invention.
FIG. 3 is a schematic view for explaining the principle that the plate-shaped material forms the card house structure, which constitutes the card house structure of the present invention.
FIG. 4 is an electron microscope photograph of a liquid drop cosmetic material according to an embodiment of the present invention.
FIG. 5 is a photograph of the skin on which a cosmetic material according to an embodiment of the present invention is applied and rubbed.

### [Best Mode]

Hereinafter, examples of the present invention will be described in detail so as to carrying out easily for those skilled in the art to which the present invention pertains, with reference to the accompanying drawings. The present invention may, however, be embodied in many different forms and should not be limited to the embodiments set forth herein. In order to clearly illustrate the present invention, parts not related to the description are omitted, and similar parts are denoted by similar reference numerals throughout the specification.

Throughout the specification, when some parts "comprise" a component, it means that it can include other components as well, without excluding other components unless specifically stated otherwise.

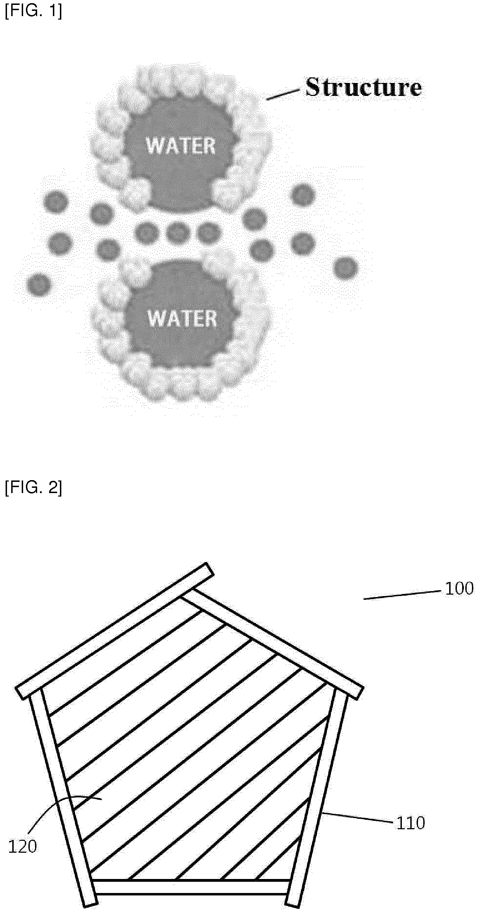

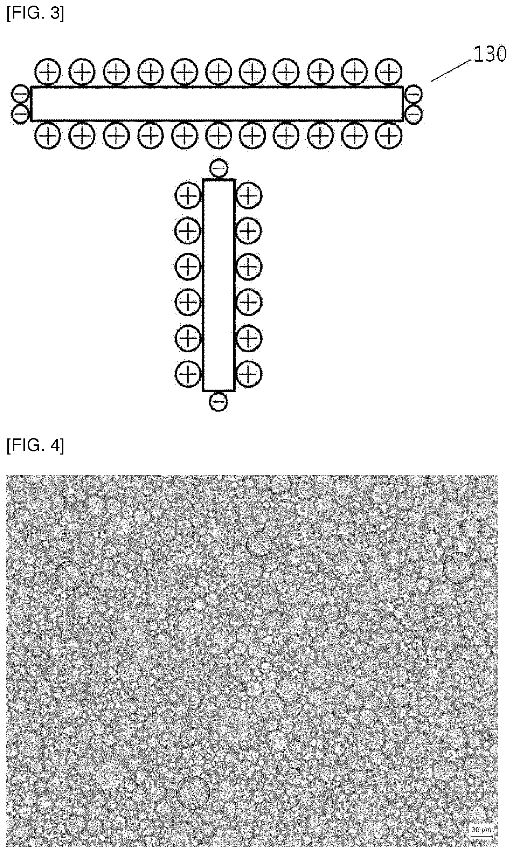

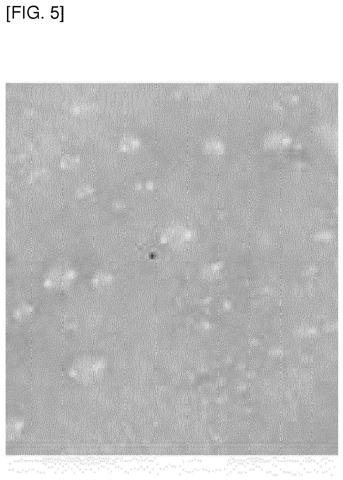

FIG. 1 is a schematic view showing that water is released from a water releasing cosmetic material. FIG. 2 is a schematic view showing that a card house structure in which a liquid drop is contained according to an embodiment of the present invention. FIG. 3 is a schematic view for explaining the principle that the plate-shaped material forms the card house structure, which constitutes the card house structure of the present invention. FIG. 4 is an electron microscope photograph of a liquid drop cosmetic material according to an embodiment of the present invention. FIG. 5 is a photograph of the skin on which a cosmetic material according to an embodiment of the present invention is applied and rubbed.

The drop (specifically, water) releasing cosmetic material may have a form which the structure receives water to the inside thereof as shown in FIG. 1 of the cosmetic material and the structure deforms by rubbing during use to release water.

A cosmetic material of present invention is liquid drop releasing cosmetic material by rubbing characterized in that it comprises: (a) a card house structure 110 having a space formed with a plate-shaped substance 130 therein; and (b) liquid drop 120 contained in the space of the card house structure 110.

The drop releasing component 100 of the present invention has the card house structure as shown in FIG. 2, in which a plate-shape substance 130 having positive electric charge on the plane and negative electric charge on the edge thereof in FIG. 3 is mixed in the cosmetic material so that the plane having positive electric charge and the edge having negative electric charge combine. The card house structure may be a 4 to 100-sided body, and preferably a 10 to 50-sided body. When the cosmetic material is within the above range, the liquid drop 120 is easily released by rubbing during the use, and the stability of the liquid drop releasing component 100 in the cosmetic material is excellent.

The plate-shaped material 130 may have positive (+) electric charge on the plane and negative (-) electric charge on the edge by the cation treatment, and preferably hectorite or bentonite modified with a cation. More preferably, disteardimonium hectorite is used. In this case, the component simultaneously functions as a thickening agent in the cosmetic material to have the remarkably excellent stability of the liquid drop releasing component 100 in the cosmetic material.

Preferably, the card house structure 110 of the present invention further comprises: at least one oil selected from the group consisting of coco-caprylate/caprate, caprylic/capric triglyceride, dicaprylyl carbonate, myristyl myristate, dicaprylyl ether, cetyl palmitate, octyldodecanol, hexyldecanol, hexyldecyl stearate, cetyl palmitate, isostearyl isostearate, decyl oleate, hydrogenated vegetable oil, methylheptyl isostearate, nigella sativa seed oil, camellia oleifera seed oil and passiflora incarnata oil; and at least one emulsifier selected from the group consisting of polyglyceryl-4 isostearate (HLB 5), polyglyceryl-3 polyricinoleate (HLB 4), polyglyceryl-3 diisostearate (HLB 5.5), polyglyceryl-4 oleate (HLB 3), polyglyceryl-4 diisostearate/polyhydroxystearate/sebacate (HLB 5), diisostearoyl polyglyceryl-3 dimer dilinoleate (HLB 5), polyglyceryl-3 polyricinoleate (and) sorbitan isostearate (HLB 4.2), glyceryl laurate (HLB 5.2), sorbitan oleate (HLB 4.3) and sorbitan sesquioleate (HLB 3.7). Specifically, the card house structure 110 comprises 5 to 20 parts by weight of a plate-shaped substance; 40 to 60 parts by weight of oil; and 30 to 50 parts by weight of an emulsifier. In this case, the liquid drop 120 is easily released by the rubbing during the use of the cosmetic material, the feeling of use is more excellent and the stability of the liquid drop releasing component 100 in the cosmetic material is more excellent.

In addition, the size of the liquid drop releasing component 100 in the cosmetic material of the present invention may be 10 to 60 µm in diameter, preferably 20 to 45 µm, more preferably 20 to 45 µm in diameter of at least 50%. In this case, the liquid drop 120 is easily released by rubbing during the use of the cosmetic material, and the stability of the liquid drop releasing component 100 in the cosmetic material is more excellent. If the size of the liquid drop releasing component 100 is too small, the liquid drop 120 may not be easily released and if the size of the liquid drop releasing component 100 is too large, the stability in the cosmetic material may be significantly reduced.

In the cosmetic material of the present invention, the liquid drop 120 may be in the form of a liquid, and specifically water, but is not limited thereto. The water may be water in which known components that can be used in cosmetic material is dissolved. Specific examples of the known components include ions, flavors, extracts, nutrients, and other functional ingredients used in a cosmetic material, which may be used in an amount of the conventional additives (0.001 to 10% by weight) and in combination with at least two

The liquid drop releasing cosmetic material according to the present invention may be embodied in a variety of formulations capable of releasing the liquid drop 120 comprising a known component. For specific example, the card house structure 110 having a space formed with a plate-shaped substance 130 therein is preferably 0.5 to 20% by weight, more preferably 1 to 10% by weight. When it is used within the above range, the liquid drop 120 is easily released by rubbing during the use of the cosmetic material and the stability of the liquid drop releasing component 100 in the cosmetic material is more excellent.

The liquid drop releasing cosmetic material according to the present invention can be prepared by mixing the material constituting the card house structure 110 with the cosmetic material containing the liquid drop 120. For a specific example, in the case of a cream, it may be prepared by mixing 3 to 20 parts by weight of the material constituting the card house structure 110 with 80 to 97 parts by weight of water in which known components used in cosmetic material are dissolved and optionally 1 to 30 parts by weight of a known ingredient that can be used in cosmetic material such as a moisturizer or a softener, etc., but is not limited thereto. In addition, the material constituting the card house structure 110 may be the plate-shaped material 130 only, and preferably the plate-shaped material 130 of 5 to 20 parts by weight; 40 to 60 parts by weight of oil; and 30 to 50 parts by weight of an emulsifier.

As a more specific example, 5% by weight of the card house structure including 10 parts by weight of distearmonium hectorite, 40 parts by weight of polyglyceryl-4 isostearate and 50 parts by weight of Coco-caprylate/caprate, 7% by weight of a moisturizing agent, 0.5% by weight of NaCl, 3% by weight of glycerin, 1.7% by weight of propanediol, 0.5% by weight of plant extract, 0.7% by weight of hexanediol, 0.03% by weight of fragrance and a residual amount of water are mixed to prepare cream. An electron microscope photograph of the cream is shown in FIG. 4 and a photograph of the skin to which the cream is applied and rubbed is shown in FIG. 5.

As shown in FIG. 4, it can be confirmed that the liquid drop 120 is contained in the card house structure 110 of the cosmetic material according to the present invention and the releasing effect of the liquid drop 120 is excellent as shown in FIG. 5.

A liquid drop 120 releasing cosmetic material according to the present invention has an excellent visual effect and feeling of use by releasing liquid drop 120 due to rubbing during use and fundamentally solves side effect and antipathy of users due to silicone polymer because it is free from a silicone polymer, and has excellent stability in a cosmetic material of a drop releasing structure 110.

Although the present invention has been described in detail with reference to the specific features, it will be apparent to those skilled in the art that this description is only for a preferred embodiment and does not limit the scope of the present invention. Thus, the substantial scope of the present invention will be defined by the appended claims and equivalents thereof.

## Claims

1. A cosmetic material of releasing liquid drop by rubbing, which comprises:
(a) a card house structure having a space formed with a plate-shaped substance therein; and
(b) liquid drop contained in the space of the card house structure.

2. The cosmetic material according to claim 1, wherein the plate-shaped substance of the (a) has an (+) electric charge on a surface thereof and an (-) electric charge in an edge thereof.

3. The cosmetic material according to claim 1, wherein the plate-shaped substance of the (a) is hectorite or bentonite modified with a cation.

4. The cosmetic material according to claim 1, wherein the plate-shaped substance of the (a) is distearmonium hectorite.

5. The cosmetic material according to claim 1, wherein the card house structure of the (a) further comprises:
at least one oil selected from the group consisting of coco-caprylate/caprate, caprylic/capric triglyceride, dicaprylyl carbonate, myristyl myristate, dicaprylyl ether, cetyl palmitate, octyldodecanol, hexyldecanol, hexyldecyl stearate, cetyl palmitate, isostearyl isostearate, decyl oleate, hydrogenated vegetable oil, methylheptyl isostearate, nigella sativa seed oil, camellia oleifera seed oil and passiflora incarnata oil; and
at least one emulsifier selected from the group consisting of polyglyceryl-4 isostearate (HLB 5), polyglyceryl-3 polyricinoleate (HLB 4), polyglyceryl-3 diisostearate (HLB 5.5), polyglyceryl-4 oleate (HLB 3), polyglyceryl-4 diisostearate/polyhydroxystearate/sebacate (HLB 5), diisostearoyl polyglyceryl-3 dimer dilinoleate (HLB 5), polyglyceryl-3 polyricinoleate (and) sorbitan isostearate (HLB 4.2), glyceryl laurate (HLB 5.2), sorbitan oleate (HLB 4.3) and sorbitan sesquioleate (HLB 3.7).
[Claim 6] The cosmetic material according to claim 5, wherein the card house structure of the (a) comprises 5 to 20 parts by weight of a plate-shaped substance; 40 to 60 parts by weight of oil; and 30 to 50 parts by weight of an emulsifier.
[Claim 7] The cosmetic material according to claim 1, wherein a size of the card house structure having liquid drop in the space therein is 10 to 60 µm in diameter.
[Claim 8] The cosmetic material according to claim 1, wherein the card house structure having a space formed with a plate-shaped substance therein of the (a) is in an amount of 0.5 to 20% by weight of cosmetic composition.
[Claim 9] The cosmetic material according to claim 1, wherein the liquid drop is water.
